# FASCICULE DE BREVET EUROPEEN

(11) **EP 0 621 776 B1**
(45) Date de publication et mention de la délivrance du brevet: **17.04.1996**
(21) Numéro de dépôt: 93904081.2
(22) Date de dépôt: 14.01.1993
(51) Int. Cl.: A61K 9/16, A61L 27/00, A61L 31/00

(54) **COMPOSITION INJECTABLE CONTENANT DES MICROCAPSULES DE COLLAGENE**
KOLLAGENMIKROKAPSELN ENTHALTENDE INJEZIERBARE ZUSAMMENSETZUNG
INJECTABLE COMPOSITION CONTAINING COLLAGEN MICROCAPSULES

(30) Priorité: 16.01.1992 FR 9200411
(43) Date de publication de la demande: 02.11.1994
(73) Titulaire: COLETICA, F-69007 Lyon (FR)
(72) Inventeur: ORLY, Isabelle, F-69002 Lyon (FR); HUC, Alain, F-69110 Ste-Foy-les-Lyon (FR)
(74) Mandataire: Portal, Gérard
(86) Numéro de dépôt international: FR9300035
(87) Numéro de publication internationale: WO9313755

(56) Documents cités:
- EP-A- 0 196 197
- EP-A- 0 230 647
- EP-A- 0 381 543
- EP-A- 0 397 542

## Description

### DOMAINE DE L'INVENTION

La présente invention a essentiellement pour objet des compositions injectables contenant en suspension des microcapsules à base de collagène, leur utilisation biomédicale et des compositions pharmaceutiques.

La présente invention a plus précisément pour objet la réalisation d'un support injectable permettant d'obtenir des comblements de pertes de substance ou d'introduire commodément des substances actives dans l'organisme d'un être vivant, en particulier d'un être humain.

### ETAT DE LA TECHNIQUE

Un grand nombre d'investigations ont été menées pour mettre au point des matériaux de comblement. En ce qui concerne le comblement des tissus mous, on peut citer :
- les injections de silicones, de gélatine, de collagène autologue et hétérologue
- l'implantation de fils (catgut, Gore-Tex, or, collagène)
Il se dessine clairement aujourd'hui que le matériau le plus performant en terme de biocompatibilité et de stabilité in vivo est le collagène (injectable et fils) hétérologue (origine bovine).

Les applications les plus courantes des matériaux de comblement des tissus mous concernent essentiellement le traitement des dépressions cutanées et pertes de substance faciales dûes à l'âge (rides), ou à d'autres causes (maladies, traumatismes, interventions chirurgicales...) et qui se traduisent par des cicatrices, atrophies, irrégularités etc...

Dans certains de ces cas, l'utilisation de fils de collagène réticulé telle que décrite dans le document n^{o} 91 04518 déposé par BIOETICA est tout particulièrement indiquée compte tenu des grandes qualités de biocompatibité, de stabilité in vivo et du pouvoir de comblement élevé de ce biomatériau.

Cependant, l'utilisation de ces fils, si elle convient parfaitement au comblement des rides profondes, s'avère moins adaptée à d'autres situations (par exemple, rides superficielles, défauts cutanés ne se présentant pas sous la forme d'un sillon). Dans ces cas, il est nécessaire de recourir à une forme injectable.

De plus, en dehors de la chirurgie esthétique, il existe des domaines médicaux (otorhinolaryngologie, urologie, orthopédie...)
pour lesquels la nature des comblements à réaliser et/ou l'accessibilité des sites d'implantation nécessite impérativement l'emploi d'une forme injectable.
- Les inventions existantes les plus récentes sont décrites dans les documents EP 83868 A1, EP-A-0196197 et EP 89145 A2 déposés par la société COLLAGEN CORPORATION EP 132979 A2 déposé par la société KOKEN CO. LTD et US-A-3 949 073.
- Les collagènes injectables existants sont des solutions collagéniques. Dans la plupart des cas, il convient que l'implant puisse être injecté au moyen d'aiguilles fines (0,3 à 0,5 mm de diamètre en général). Pour répondre à cet impératif, les solutions doivent être relativement fluides donc peu concentrées (2 à 6,5 %). Cette limitation pose le problème de la biodégradabilité et de la tenue dans le temps de tels implants, les quantités de matières actives administrées étant relativement faibles.
- Pour des raisons de solubilité et de biocompatibilité, le collagène utilisé est un atélocollagène, c'est-à-dire un collagène duquel ont été éliminées les régions termina les de structure non-hélicoïdale dénommées télopeptides. Ces télopeptides sont en effet le siège de liaisons intra- et intermoléculaires de type liaisons croisées qui rendent le collagène insoluble. De plus, les télopeptides sont porteurs des principaux déterminants antigéniques du collagène. Dans les produits existants, l'atélocollagène est obtenu par traitement protéasique à la pepsine qui attaque préférentiellement les télopeptides sans détruire la structure hélicoïdale. La pepsine présente cependant l'inconvénient de se fixer très fortement à son substrat et donc d'être difficilement éliminable. Or il s'agit d'une molécule fortement antigénique dont la présence, même en faible quantité, risque d'altérer la biocompatibilité du produit ainsi obtenu.
- Pour assurer sa fonction de comblement, il convient que l'implant conserve, après l'injection, un certain volume et qu'il ne diffuse pas de façon importante dans les tissus environnants. Il constitue ainsi une sorte de matrice qui, maintenue pendant un temps suffisant, est colonisée par les tissus de l'hôte et permet une restauration de la zone traitée. Dans les produits existants, cette caractéristique est satisfaite par l'utilisation de solutions d'atélocollagène qui précipitent à la température du corps, conduisant donc à la formation de fibres qui subsistent au site d'injection alors que l'excipient est progressivement résorbé (voir US-A-3 949 073).

Il résulte de ceci qu'avant l'injection le produit doit être impérativement conservé à basse température (inférieure à 10^{o}C) de façon à ce qu'il conserve sa fluidité, propriété nécessaire à son administration par injection. En conséquence, les collagènes injectables basés sur ce principe nécessitent des précautions particulières de conservation qui en compliquent l'utilisation.
- La limitation majeure des collagènes injectables antérieurs réside dans leur résorbabilité "in vivo".

En effet, la dégradation du collagène est un processus biologique normal faisant partie du métabolisme des tissus conjonctifs. Ce processus fait intervenir un certain nombre d'enzymes, en particulier les collagénases qui sont responsables de l'attaque initiale du collagène. La biodégradabilité du collagène pose un problème particulièrement important. En effet, le comblement des dépressions et pertes de substance doit être un traitement définitif ou en tout cas raisonnablement durable ce qui s'accompagne mal d'une résorption rapide de l'implant.

Il existe différents traitements physiques et chimiques susceptibles de diminuer la résorbabilité en augmentant le nombre de liaisons de réticulation entre les molécules de collagène. Le glutaraldéhyde est l'agent réticulant le plus largement employé (comme décrit dans EP-A-0 089 145).

Il est particulièrement efficace mais présente l'inconvénient majeur de se polymériser en solution. Ainsi, les matériaux réticulés par cet agent libèrent au cours du temps des monomères de glutaraldéhyde, cytotoxiques au-delà de 10-25 ppm. Les collagènes injectables fabriqués selon ce principe (brevet EP 89 145 A2) sont donc en général moins dégradables que les implants non réticulés (stabilité moyenne de 1 à 2 ans au lieu de 6 mois à 1 an, en ce qui concerne les comblements de rides). En contrepartie, ils peuvent provoquer des réactions adverses plus fréquentes ce qui suscite une certaine méfiance et une utilisation moindre.

### RESUME DE L'INVENTION

La présente invention a pour but principal de fournir une solution permettant de réaliser un matériau injectable pouvant être aisément injecté même à l'aide d'aiguille fine dont le diamètre est de l'ordre de 0,3 à 0,5 mm, avec une teneur élevée en collagène, en particulier d'au moins 7 % en poids/volume et de préférence aussi élevé qu'environ 10 %.

La présente invention a encore pour but de résoudre le nouveau problème technique énoncé ci-dessus en fournissant une solution qui permette de fournir un système de vectorisation de substances actives en permettant l'optimisation thérapeutique des molécules actives, par exemple par libération dans l'organisme de façon contrôlée, ciblage des tissus ou organes visés, et protection de l'actif.

La présente invention a encore pour but de résoudre le nouveau problème technique énoncé ci-dessus, en fournissant une solution de meilleure biocompatibilité dont les produits de dégradation ne donnent pas naissance à des résidus pouvant provoquer des réactions indésirables.

La présente invention a encore pour but de résoudre les nouveaux problèmes techniques énoncés ci-dessus à l'aide d'une solution qui permette de moduler les vitesses de dégradation du matériau injecté dans un être vivant, en particulier un être humain de manière à pouvoir régler la durée de vie d'un implant et également la cinétique de libération d'un actif véhiculé.

L'invention a aussi pour but de fournir des matériaux de comblement dont la stabilité in vivo peut être au moins égale à celle des matériaux collagéniques traités au glutaraldéhyde.

Tous ces problèmes techniques sont résolus simultanément, de manière satisfaisante, sûre, fiable et reproductible, utilisable à l'échelle industrielle et médicale, tout en étant également peu coûteuse.

Ainsi, selon un premier aspect, la présente invention fournit une composition à base de collagène, aisément injectable, caractérisée en ce qu'elle comprend des microcapsules d'atélocollagène ou d'un mélange d'atélocollagène et d'un polysaccharide, en particulier un glycosaminoglycanne, en suspension dans une solution support biocompatible visqueuse. De préférence, l'ensemble des microcapsules et de la solution support visqueuse a une viscosité permettant l'injection de ladite composition, en particulier sous la forme d'un fil continu à la sortie d'une aiguille.

Selon une variante de réalisation particulière, la solution support comprend au moins une substance protéique, par exemple de l'atélocollagène et/ou une substance polysaccharidique.

Lorsque la solution support comprend une substance protéique, celle-ci est avantageusement de l'atélocollagène, qui peut être obtenu par voie enzymatique ou par voie chimique, de préférence obtenu par traitement chimique notamment à la soude, le traitement chimique étant particulièrement avantageux puisqu'il permet d'éviter toute présence résiduelle d'enzyme comme c'est le cas dans le cadre de l'utilisation d'un traitement enzymatique, habituellement la pepsine, ce qui augmente la biocompatibilité du matériau. Dans les cas d'obtention d'atélocollagène par traitement enzymatique notamment à la pepsine, ou traitement chimique, notamment à la soude, il est possible de moduler le degré de déréticulation du collagène et donc la viscosité du matériau support.

Selon une autre variante de réalisation particulière, la substance polysaccharidique est utilisée, éventuellement en mélange avec la substance protéique dans le matériau support, et choisie parmi le groupe consistant d'un glycosaminoglycanne, d'un alginate, d'un dextran, d'une cellulose ou d'un dérivé de cellulose, d'une gomme xanthane, d'une gomme arabique, ainsi que leur(s) mélange(s).

Ainsi, selon une variante de réalisation avantageuse, l'atélocollagène des microcapsules est déréticulé avec un taux de déréticulation adapté à l'application souhaitée.

Comme polysaccharide qui peut être utilisé en mélange avec l'atélocollagène pour la préparation des microcapsules précitées, on peut utiliser en particulier un glycosaminoglycanne choisi parmi le groupe consistant des glycosaminoglycannes de structure choisis parmi le chondroïtine-4-sulfate, le chondroïtine-6-sulfate, le dermatane-sulfate, l'héparane-sulfate, le kératane-sulfate, ainsi que l'héparine et ses dérivés, en particulier les hérapines de bas poids moléculaire ayant un poids moléculaire compris entre environ 2 000 et environ 10 000 ; et un polyholoside, et en particulier le dextran.

Le procédé de préparation des microcapsules est basé sur celui décrit dans un brevet antérieur du déposant FR-A-2 642 329 qui est incorporé ici par référence.

Il est avantageux que la proportion de polysaccharide relativement à l'atélocollagène soit comprise entre 10 et 70 %, mieux entre 15 et 50 % en poids.

Dans le cadre de la préparation des microcapsules, on réalise une réticulation de l'atélocollagène et éventuellement des polysaccharides par réticulation interfaciale en réalisant une émulsion de la solution d'atélocollagène et de polysaccharides comme phase dispersée dans un liquide hydrophobe formant la phase continue, dans lequel l'atélocollagène et/ou les polysaccharides sont essentiellement insolubles.

Pour la réticulation, on ajoute à l'émulsion de la solution d'atélocollagène et éventuellement des polysaccharides, un agent réticulant comportant des groupes réactifs capables de réagir avec les groupes acylables de l'atélocollagène et des polysaccharides de manière à réaliser une réaction de réticulation interfaciale. Comme agent réticulant préféré, on utilise un dichlorure d'acide ou un anhydride d'acide ou un acide carboxylique di- ou poly-basique. Comme agents réticulants préférés, on utilise le chlorure de téréphtaloyle, le chlorure d'acide phtalique, le chlolure d'acide sébacique, le chlorure d'acide succinique, le chlorure d'un acide tricarboxylique comme l'acide citrique ou un anhydride d'acide comme l'anhydride succinique.

Comme liquide hydrophobe, dans lequel l'atélocollagène et/ou les polysaccharides sont insolubles, on utilise de préférence des esters d'acide gras, de préférence choisis parmi ceux habituellement administrables par voie parentérale tels que triglycérides, oléate d'éthyle ; ou dépourvus de propriétés toxiques, tels que le myristate d'isopropyle, le cocoate d'éthyle 2-hexyle.

La concentration en polysaccharides dans la solution de polysaccharides est avantageusement comprise entre 0,5 et 4 %, encore mieux entre 0,5 et 2 %, et encore de préférence est voisine de 1 %.

La solution d'atélocollagène pour la préparation des microcapsules est une solution aqueuse d'atélocollagène ayant une concentration comprise entre 0,5 et 2 % en poids.

Pour la préparation des microcaspules, l'atélocollagène peut avoir été obtenu par digestion enzymatique de collagène, notamment à la pepsine, ou encore par traitement chimique à la soude.

Pour le procédé de préparation proprement dit des microcapsules, on se reportera au document FR-A-2 642 329 qui est incorporé ici par référence, et en particulier aux exemples 1 à 6.

Selon un mode de réalisation avantageux, la dimension des microcapsules est inférieure ou égale à environ 500 µm. Dans certains cas, la dimension des microcapsules est inférieure à 250 µm et peut être même comprise entre 20 et 50 µm, en particulier dans le cas où l'on désire injecter la composition par une aiguille fine ayant un diamètre comprise entre 0,3 et 0,5 mm.

Selon une variante particulière, la proportion relative des microcapsules par rapport àla solution support biocompatible visqueuse est comprise entre 0,5-15/99,5-85 en poids/volume.

Selon un mode de réalisation particulièrement avantageux, les microcapsules contiennent au moins une substance active, soit encapsulée à l'intérieur, soit dans la masse des parois des microcapsules, soit greffée en surface interne et/ou externe. Cette substance active peut être biologiquement active, cosmétiquement ou pharmaceutiquement active. Par substance biologiquement active, on entend une substance qui a un effet biologique sur l'organisme qui n'est pas nécessairement cosmétique ou thérapeutique. Une substance active actuellement préférée est l'hydroxyapatite, en particulier sous forme de particules, par exemple de taille égale à environ 50 µm.

Selon un deuxième aspect, la présente invention fournit également une utilisation de la composition précitée comme agent vecteur de substance biologiquement, cosmétiquement ou pharmaceutiquement active.

La présente invention couvre aussi une composition pharmaceutique, caractérisée en ce qu'elle comprend une composition à base de collagène aisément injectable telle que précédemment définie.

L'invention couvre enfin un implant, caractérisé en ce qu'il a été préparé à partir d'une composition injectable précédemment définie.

D'autre part, l'invention couvre également des réalisations particulières de compositions. Comme réalisations particulières, on préfère une réalisation selon laquelle les microcapsules sont à base d'atélocollagène éventuellement mélangé avec un glycosaminoglycanne comme le chondroïtine-4-sulfate, ces microcapsules contenant des granules d'hydroxyapatite en suspension dans une solution support biocompatible visqueuse d'un gel d'atélocollagène éventuellement mélangé avec un glycosaminoglycanne, en particulier le chondroïtine-4-sulfate. Lorsque les granules d'hydroxyapatite ont une dimension d'environ 50 µm, les microcapsules ont une dimension de l'ordre de 200 µm.

Selon un mode de réalisation particulièrement avantageux, la solution support biocompatible visqueuse est adaptée aux conditions physiologiques de pH et de pression osmotique, et en ayant de préférence un pH compris entre 6,8 et 7,5 et une pression osmotique comprise entre 260 et 320 mOsm/l H₂O. On peut obtenir un tel pH et une telle osmolarité de la solution support en ajustant avec un tampon phosphate comprenant par exemple un mélange de phosphates, de sodium et/ou de potassium mono- et disodiques, ou d'un mélange tampon phosphate et chlorure de sodium pour compléter la solution support de suspension des microcapsules.

Grâce à la nature des constituants des microcapsules, atélocollagène éventuellement mélangé à des polysaccharides, les microcapsules sont souples, déformables, de sorte qu'en suspension dans la solution support biocompatible visqueuse selon l'invention, ta composition possède dans son ensemble des propriétés d'écoulement telles qu'on peut injecter aisément au moyen d'une aiguille fine ayant un diamètre de 0,3 à 0,5mm , un matériau contenant jusqu'à 10 % de collagène en poids/volume. Cette possibilité représente une augmentation de 35 % par rapport aux produits antérieurs les plus concentrés (6,5 %).

Ainsi, les compositions selon l'invention sont particulièrement appropriées pour être utilisées comme matériaux de comblements, soit pour réaliser des comblements de tissus mous, ou des comblements osseux pour la reconstruction osseuse. Dans ces applications, il est évident que toute augmentation de la concentration en substance active augmente de facto la durée de vie de l'implant et l'efficacité du comblement.

La composition selon l'invention conjugue ainsi les propriétés biologiques du collagène, sous sa forme atélocollagène, à celles des autres substances éventuellement mises en oeuvre (polysaccharides co-réticulés avec l'atélocollagène, substances actives en solution ou en suspension dans les sphères, molécules greffées à leur surface).

Ainsi, les compositions selon l'invention possèdent un vaste champ d'application en tant que système de vectorisation de substances actives. Cette forme galénique est particulièrement intéressante puisqu'elle permet, selon les utilisations :
- l'optimisation thérapeutique de molécules actives par libération dans l'organisme de façon contrôlée, ciblage des tissus ou organes visés, protection de l'actif ;
- l'accès par voie percutanée à des sites habituellement accessibles uniquement par voie chirurgicale (site osseux par exemple), ce qui limite le traumatisme subi par le patient et les coûts et durée d'hospitalisation.

Il est bien entendu que l'on peut également envisager un certain nombre d'applications dans lesquelles peuvent être mises à profit à la fois la fonction de comblement et l'utilisation d'une forme galénique particulièrement avantageuse.

La composition selon l'invention est particulièrement adaptée à l'utilisation en tant que vecteur dans la mesure où sa dégradation ne donne pas naissance à des résidus pouvant provoquer des réactions indésirables. Ceci constitue un avantage majeur par rapport à certains polymères synthétiques de la technique antérieure.

La dégradation des microcapsules peut être modulée dans de très larges limites d'une part par un degré de déréticulation variable de l'atélocollagène par voie chimique ou enzymatique ; et d'autre part en réglant le taux de réticulation interfaciale. On peut ainsi régler la vitesse de résorbabilité depuis une vitesse rapide à une stabilité supérieure à celle obtenue avec des matériaux réticulés au glutaraldéhyde, tout en évitant les inconvénients liés à la toxicité de cet agent.

On multiplie ainsi le champ d'application des compositions selon l'invention, en jouant sur la durée de vie de la composition, en particulier sous forme d'implant, selon qu'elle est destinée à subsister le plus longtemps possible au niveau du site d'implantation ou être résorbée et remplacée plus ou moins rapidement par les tissus de l'hôte ; et la cinétique de libération de l'actif.

On comprend ainsi que la présente invention fournit une solution technique présentant des avantages techniques déterminant par rapport à l'état de la technique antérieure.

Dans les exemples, tous les pourcentages sont donnés en poids, sauf indication contraire.

### Exemple 1 : Suspension de microcapsules d'atélocollagène de 20 à 50 µm dans un gel d'atélocollagène

Cette suspension constitue un produit destiné à assurer une fonction de comblement, des tissus mous en particulier.

### a) Préparation de l'atélocollagène par traitement à la pepsine

La peau de veau fraîchement abattu est soumise à épilation chimique dans un bain contenant 3 % de sulfure de sodium et 4 % de chaux, la proportion étant de 100 g de peau pour 200 ml de solution. Le derme est alors isolé du reste de la peau par une opération de refendage grâce à une scie tournante à ruban.

Le tissu obtenu est broyé et extrudé à travers une grille comportant des trous de 4 mm.

Le broyat est dispersé dans une solution d'acide acétique 0,5 M contenant la pepsine dans les proportions de 1 g d'enzyme pour 10 g de collagène sec, le pH du mélange étant ajusté si nécessaire pour obtenir un pH compris entre 2 et 3. Après agitation, le mélange est laissé en incubation pendant 36 h à 10^{o}C. Après filtration, le collagène est précipité par addition de NaCl 0,7 M. Après agitation et incubation (4 h), les fibres précipitées sont récupérées par centrifugation (14 000 tr/min). Le surnageant est éliminé et la pepsine est inactivée par dispersion du culot dans un tampon Tris 0,05 M - NaCl 0,15 M dont le pH est ajusté, après dispersion des fibres, à 8,5. Après 3 jours d'incubation à 10^{o}C, la dispersion est dialysée contre de l'eau désionisée et stérile à l'aide de membranes de dialyse, de préférence formées par des boyaux dont le seuil de coupure est compris entre 6 000 et 8 000 daltons.

### b) Préparation de la solution d'atélocollagène en milieu tamponné à pH 8-8,2

L'atélocollagène provenant des boyaux de dialyse est diluée par une solution de bicarbonate de sodium de volume et de concentration tels que les concentrations finales soient les suivantes :

| | |
|---|---|
| - Atélocollagène | 1 % |
| - Bicarbonate de sodium anhydre | 4 % |

Il est vérifié que le pH de la solution obtenue est bien compris entre 8 et 8,2. On prépare 1 kg de cette solution.

### c) Préparation de l'agent de réticulation

100 g de chlorure de téréphtaloyle sont ajoutés à 4 l d'un solvant hydrophobe, préférentiellement des esters d'acides gras choisis parmi ceux habituellement administrables par voie parentérale (triglycérides, oléate d'éthyle par exemple) ou dépourvus de propriétés toxiques (myristate d'isopropyle, cocoate d'éthyle-2-hexyle par exemple). L'ensemble est agité par agitation mécanique.

### d) Emulsification

3 l du solvant hydrophobe choisi selon les critères cités au-dessus sont introduits dans un récipient. La solution d'atélocollagène tamponnée préparée précédemment est ajoutée sous agitation à l'Ultra Turax (R) tournant à 7 200 tr/min.

### e) Réticulation

La solution d'agent réticulant est ajoutée à l'émulsion obtenue. L'agitation est maintenue pendant 30 min.

On obtient ainsi des microcapsules.

### f) Lavages

Trois lavages sont effectués par 10 l du solvant hydrophobe et les microcapsules sont recueillies par décantation à l'aide d'une décanteuse Robatel tournant à 1 000 tr/min.

Cinq à huit lavages à l'éthanol (15 à 20 l au total) sont effectués de la même façon, suivis de trois à cinq lavages à l'eau désionisée et stérile.

### g) Lyophilisation

On procède à une lyophilisation de manière classique du produit lavé contenant les microcapsules.

### h) Stérilisation

Après conditionnement des sphères sèches sous emballage scellé, les microcapsules sont stérilisées par exposition au rayonnement gamma (25 kGy).

### i) Préparation du milieu de suspension

L'atélocollagène est déréticulé par traitement à la soude dans les conditions exposées ci-dessous.

Après épilation, refendage et broyage des peaux tels que décrits en a), le broyat est soumis à deux lavages au tampon phosphate pH 7,8 (21,7 g/l de Na₂HPO₄ et 0,78 g/L de KH₂PO₄) puis à deux lavages à l'eau désionisée et stérile.

Le broyat est alors mis en contact pendant huit jours avec une solution de soude à raison de 1 kg de broyat pour 4 l de solution de concentration telle que la quantité finale de soude soit de 8 % (p/v). A l'issue de ce traitement, l'atélocollagène est précipité par addition d'acide chlorydrique concentré jusqu'à l'obtention d'un pH compris entre 2 et 2,5. La suspension obtenue est introduite dans des boudins de dialyse (seuil de coupure compris entre 6 000 et 8 000 daltons) et dialysée contre de l'eau stérile et désionisée.

L'atélocollagène obtenu est ensuite lyophilisé, stérilisé par rayonnement gamma et mis en solution dans des conditions stériles. Le pH et l'osmolarité de la solution sont ajustés par utililisation d'un tampon phosphate (mélange de phosphates de sodium et/ou de potassium mono- et disodique) ou d'un mélange tampon phosphate et chlorure de sodium de façon à obtenir un pH compris entre 6,8 et 7,5 et une pression osmotique comprise entre 260 et 320 mOsm/l H₂O.

Exemple de composition du milieu de suspension :

| | |
|---|---|
| - Atélocollagène | 1 % |
| - Na₂HPO₄ | 0,02 M |
| - NaCl | 0,13 M |
| - pH 7,3 | |

### j) Mise en suspension

Les microcapsules sont mises en suspensions dans le milieu de suspension d'atélocollagène obtenu à l'étape i) dans des conditions stériles à raison de 100 mg de sphères sèches par ml de milieu de suspension. La suspension est agitée de façon à obtenir un mélange parfaitement homogène. Le pH du mélange est vérifié.

### k) Préparation des seringues

Le mélange est introduit dans des conditions stériles, dans des seringues de 1 ml munies d'un embout de type luer-lock et d'aiguilles de diamètre de 0,3 à 0,5 mm.

### l) Evaluation du produit obtenu

* Stabilité thermique par analyse en Calorimétrie Différentielle Programmée :
Les paramètres enregistrés sur les microcapsules après lyophilisation sont les suivants :
- température de début de dénaturation : 65^{o}C
- température de pic de dénaturation : 72,6^{o}C
- température de fin de dénaturation : 80^{o}C
Ces valeurs sont particulièrement élevées et sont du même ordre de grandeur qu'avec les produits obtenus par réticulation au glutaraldéhyde par exemple.
* Evaluation in vivo :
Les essais ont été réalisés sur des rats Wistar femelles de 180 à 200 g.

Le matériau est implanté sur le dos des rats, en position sous-cutanée. Chaque site d'implantation (2 par rat) reçoit environ 0,1 ml de la suspension.

Les biopsies et les contrôles histologiques effectués après 1 et 7 jours d'implantation montrent :
- un pouvoir de comblement important
- une parfaite biocompatibilité
- une absence totale de dégradation de l'implant.

Ces résultats assurent à l'implant une stabilité à long terme particulièrement élevée.

### EXEMPLE 2 : Suspension de microcapsules d'atélocollagène et de chondroïtine-4-sulfate de 200 µm environ contenant des granules d'hydroxyapatite dans un gel d'atélocollagène et de chondroïtine-4-sulfate

Dans cet exemple, des granules d'hydroxyapatite (minéral de composition chimique proche du minéral osseux et utilisé pour les reconstructions osseuses) sont incorporés dans des microcapsules. Ce système permet de véhiculer les granules d'hydroxyapatite et les rend injectables à travers un trocard. Elles pourront ainsi être implantées en site osseux par voie percutanée.

### a) Préparation de l'atélocollagène par traitement à la soude

Cette étape est réalisée dans les mêmes conditions que celles décrites pour l'exemple 1, paragraphe i).

### b) Préparation de la chondroïtine-4-sulfate

Des cloisons nasales d'agneau débarrassées des tissus musculaires et adipeux sont hachés et broyés par extrusion à travers une grille comportant des trous de 4 mm ; le broyat est placé pendant 24 heures à une température de 6^{o}C dans un tampon de chlorure de potassium (11,8 g/l de KCl ; 78,8 mg/l de cystéine, 180 mg/l d'EDTA) renfermant 1 % de papaïne "MERCK". La proportion étant de 130 g de broyat pour 1 l de tampon.

Le surnageant est séparé du culot par centrifugation en continu à l'aide d'une décanteuse tournant à 4 000 tr/min. Au surnageant sont alors ajoutés 40 g/l d'acide trichloracétique. Le précipité est éliminé par centrifugation en continu selon la technique précédente. Le surnageant est neutralisé à l'aide de soude en pastilles. Le mélange est alors dialysé contre de l'eau désionisée et stérile à l'aide de boyaux dont le seuil de coupure est compris entre 6 et 8 000 daltons. La solution dialysée est lyophilisée. La chondroïtine-4-sulfate est obtenue à l'état sec.

### c) Préparation de la solution d'atélocollagène et de chondroïtine-4-sulfate dans un milieu tamponné à pH 8,9

L'atélocollagène sous forme de fibres provenant des boyaux de dialyse est dilué par une solution contenant du chondroïtine-4-sulfate et du carbonate de sodium de volume et de concentration tels que les concentrations finales soient les suivantes :
- Atélocollagène : 1,6 %
- Chondroïtine-4-sulfate : 0,6 %
- Carbonate de sodium anhydre : 4,8 %
Le pH de l'ensemble est ajusté à 8,9 par l'acide chlorydrique concentré.

On prépare 1 kg de cette solution.

### d) Incorporation de l'hydroxyapatite

L'hydroxyapatite sous forme de granules sphériques de 50 µm de diamètre environ est incorporée dans la préparation précédemment décrite à raison de 5 % (p/v).

### e) Préparation de l'agent de réticulation

180 g de chlorure de téréphtaloyle sont ajoutés à 4 l de myristate d'isopropyle. L'ensemble est agité par agitation mécanique.

### f) Emulsification

150 ml de Span 85 (ICI Company) sont mélangés à 3 l de myristate d'isopropyle. Dans te mélange agité mécaniquement est versée la solution collagénique contenant l'hydroxyapatite. L'ensemble est agité quelques minutes pour obtenir l'émulsion.

### g) Réticulation

La solution d'agent réticulant est ajoutée à l'émulsion obtenue. L'agitation est maintenue pendant 30 minutes.

On obtient ainsi les microcapsules contenant les granulés d'hydroxyapatite.

### h) Lavages

Les microcapsules sont lavées dans les mêmes conditions que celles décrites dans l'exemple 1.

### i) Préparation du milieu de suspension

L'atélocollagène provenant des boyaux de dialyse est dilué par une solution de chondroïtine-4-sulfate de volume et de concentration tels que les concentrations finales soient les suivantes :

| | |
|---|---|
| - Atélocollagène | 0,8 % |
| - Chondroïtine-4-sulfate | 0,2 % |

Le mélange est amené à pH 8.

### j) Mise en suspension des microcapsules

Les microcapsules sont mises en suspension dans le milieu décrit ci-dessus dans des proportions telles que dans le mélange final 90 % de l'ensemble atélocollagène - chondroïtine-4-sulfate provienne des microcapsules et 10 % provienne du milieu de suspension.

On obtient ainsi une composition selon l'invention qui peut être utilisée telle quelle ou être traitée comme décrit ci-après.

### k) Lyophilisation

On lyophilise la composition obtenue à l'étape j de manière classique.

### l) Stérilisation

Mêmes conditions que l'exemple 1.

### m) Réhydratation en milieu tamponné

Le lyophilisat est mis en suspension dans des conditions stériles à raison de 500 mg de lyophilisat par ml de tampon phosphate 0,1 M. Le pH du tampon phosphate est fixé de façon à ce que le pH du mélange soit compris entre 7 et 7,3.

### n) Préparation des seringues

Le mélange est introduit, dans des conditions stériles, dans des seringues de 5 ml munies d'aiguilles de 1,5 mm de diamètre.

## Revendications

1. Composition à base de collagène, aisément injectable, caractérisée en ce qu'elle comprend des microcapsules d'atélocollagène ou d'un mélange d'atélocollagène et d'un polysaccharide, eu particulier un glycosaminoglycanne, en suspension dans une solution support biocompatible visqueuse, l'ensemble des microcapsules et de la solution support visqueuse ayant une viscosité permettant l'injection de ladite composition avec une aiguille.

2. Composition selon la revendication 1, caractérisée en ce que la solution support comprend au moins une substance protéique par exemple l'atélocollagène, et/ou une substance polysaccharidique.

3. Composition selon la revendication 1 ou 2, caractérisée en ce que la solution support comprend une substance protéique choisie parmi l'atélocollagène obtenu de préférence par traitement chimique notamment à la soude.

4. Composition selon l'une des revendications 1 à 3, caractérisée en ce que l'atélocollagène des microcapsules est déréticulé avec un taux de déréticulation adapté à l'application souhaitée.

5. Composition selon l'une des revendications 2 à 4, caractérisée en ce que la substance polysaccharidique de la solution support est choisie parmi un glycosaminoglycanne, un alginate, un dextran, une cellulose ou un dérivé de cellulose, une gomme xanthane, une gomme arabique et leur(s) mélange(s).

6. Composition selon l'une des revendications 1 à 5, caractérisée en ce que les microcapsules sont réalisées à partir d'atélocollagène ou d'un mélange d'atélocollagène et de polysaccharide réticulés, le polysaccharide étant de préférence choisi parmi un glycosaminoglycanne de structure choisi parmi le groupe consistant de chondroïtine-4-sulfate, de chondroïtine-6-sulfate, de dermatane-sulfate, d'héparane-sulfate, de kératane-sulfate, ainsi que d'héparine et ses dérivés en particulier des héparines de bas poids moléculaire ayant un poids moléculaire compris entre environ 2 000 et environ 10 000, et un polyholoside, en particulier le dextran.

7. Composition selon l'une des revendications 1 à 6, caractérisée en ce que la proportion relative en poids/volume des microcapsules par rapport à la solution support est comprise entre 0,5 et 15 % en poids de microcapsules par rapport au volume de la solution support.

8. Composition selon l'une des revendications 1 à 7, caractérisée en ce que la proportion en poids dans les microcapsules de polysacharrides par rapport à l'atélocollagène est comprise entre 10 et 70 %.

9. Composition selon l'une des revendications 1 à 8, caractérisée en ce que les microcapsules contiennent au moins une substance active, soit encapsulée à l'intérieur des microcapsules, soit dans la masse des parois, soit greffée en surface interne et/ou externe, cette substance active étant de préférence une substance biologiquement, cosmétiquement ou pharmaceutiquement active.

10. Composition selon l'une des revendications précédentes, caractérisée en ce que la dimension des microcapsules est inférieure à environ 500 µm.

11. Composition selon l'une des revendications 1 à 10, caractérisée en ce que les microcapsules sont réalisées à partir d'atélocollagène ou d'un mélange d'atélocollagène et d'un glycosaminoglycanne, en particulier du chondroïtine-4-sulfate, et contiennent des granules d'hydroxyapatite ; et la solution support est réalisée à partir d'un gel d'atélocollagène éventuellement mélangé avec un glycosaminoglycanne, en particulier le chondroïtine-4-sulfate.

12. Composition selon l'une des revendications 1 à 11, caractérisée en ce que la teneur en collagène de ladite composition est d'au moins 7% en poids/volume et de préférence d'environ 10% en poids/volume.

13. Composition selon l'une des revendications 1 à 12, caractérisée en ce que la dégradation de microcapsules est modulée en réglant le taux de réticulation interfaciale et/ou par un degré de déréticulation variable d'atélocollagène par voie chimique ou enzymatique.

14. Composition selon l'une des revendications 1 à 13, caractérisée en ce que la solution support biocompatible visqueuse précitée est adapté aux conditions physiologiques de pH et de pression osmotiques, en ayant de préférence un pH compris entre 6,8 et 7,5 et une pression osmotique comprise entre 260 et 320 mOsm/lH2O, en particulier en ajustant avec un tampon phosphate ou un mélange tampon phosphate et chlorure de sodium.

15. Composition selon l'une des revendications 1 à 14, caractérisée en ce que l'ensemble des microcapsules et la solution support visqueuse présentent une viscosité permettant l'injection de ladite composition sous la forme d'un fil continu à la sortie d'une aiguille même fine ayant un diamètre de l'ordre de 0,3 à 0,5 mm.

16. Utilisation de la composition telle que définie selon l'une quelconque des revendications 1 à 15, comme agent vecteur de substance active, en particulier de substance biologiquement, cosmétiquement ou pharmaceutiquement active.

17. Utilisation de la composition selon l'une quelconque des revendications 1 à 15, comme matériau de comblement, en particulier de tissus mous ou de tissus osseux, en particulier avec une stabilité in vivo au moins égale à celle des matériaux collagéniques traités au glutaraldéhyde.

18. Composition pharmaceutique, caractérisée en ce qu'elle comprend une composition à base de collagène telle que définie à l'une quelconque des revendications 1 à 15.

19. Matériau de comblement, caractérisé en ce qu'il a été préparé à partir d'une composition à base de collagène, telle que définie à l'une quelconque des revendications 1 à 15.

## Claims

1. Readily injectable collagen-based composition, characterized in that it comprises microcapsules of atelocollagen with a polysaccharide, in particular a glycosaminoglycan, in suspension in a viscous biocompatible carrier solution, with the microcapsules together with the viscous carrier solution having a viscosity enabling said composition to be injected from a needle.

2. Composition according to claim 1, characterized in that the carrier solution comprises at least a protein substance such as atelocollagen, and/or a polysaccharide substance.

3. Composition according to claim 1 or 2, characterized in that the carrier solution comprises a protein substance selected among the atelocollagen preferably obtained by chemical treatment, notably with soda.

4. Composition according to one of claims 1 to 3, characterized in that the atelocollagen of the microcapsules is decrosslinked with a decrosslinking rate adapted to the proposed application.

5. Composition according to one of claims 2 to 4, characterized in that the polysaccharide substance of the carrier solution is selected among a glycosaminoglycan, an alginate, a dextran, a cellulose or a cellulose derivative, a xanthan gum, a gum arabic and a mixture or mixtures thereof.

6. Composition according to one of claims 1 to 5, characterized in that the microcapsules are produced from atelocollagen or from a mixture of atelocollagen and polysaccharide crosslinked, the polysaccharide being preferably selected among a structural glycosaminoglycan selected among the group consisting of chondroitin-4-sulfate, chondroitin-6-sulfate, dermatan-sulfate, heparan-sulfate, keratan-sulfate, as well as heparin and its derivatives in particular heparins of low molecular weight having a molecular weight comprised between about 2,000 and about 10,000 and a polyholoside, in particular dextran.

7. Composition according to one of claims 1 to 6, characterized in that the relative proportion by weight/volume of the microcapsules with respect to the carrier solution is comprised between 0.5 and 15% by weight of microcapsules with respect to the volume of the carrier solution.

8. Composition according to one of claims 1 to 7, characterized in that the proportion by weight of polysaccharides in the microcapsules with respect to the atelocollagen is comprised between 10 and 70%.

9. Composition according to one of claims 1 to 8, characterized in that the microcapsules contain at least an active substance which is, either encapsulated within, or in the mass of the walls of the microcapsules, or grafted on the inner and/or outer surface, said active substance being preferably a biologically, cosmetically or pharmaceutically active substance.

10. Composition according to one of the preceding claims, characterized in that the size of the microcapsules is less than about 500 µm.

11. Composition according to one of claims 1 to 10, characterized in that the microcapsules are produced from atelocollagen or from a mixture of atelocollagen with a glycosaminoglycan, in particular chondroitin-4-sulfate, and they contain granules of hydroxyapatite; and the carrier solution is produced from a gel of atelocollagen optionally mixed with a glycosaminoglycan, in particular chondroitin-4-sulfate.

12. Composition according to one of claims 1 to 11, characterized in that the collagen content of said composition is at least 7% by weight/volume and preferably about 10% by weight/volume.

13. Composition according to one of claims 1 to 12, characterized in that the degradation of microcapsules is modulated by adjusting the rate of interfacial crosslinking and/or by a variable rate of atelocollagen decrosslinking by chemical or enzymatic means.

14. Composition according to one of claims 1 to 13, characterized in that said viscous biocompatible carrier solution is adapted to the physiological conditions of pH and osmotic pressure, preferably having a pH comprised between 6.8 and 7.5 and an osmotic pressure comprised between 260 and 320 mOsm/l H₂O, particularly by adjusting with a phosphate buffer or a mixture of phosphate buffer and sodium chloride.

15. Composition according to one of claims 1 to 14, characterized in that the microcapsules together with the viscous carrier solution have a viscosity enabling sad composition to be injected in the form of a continuous thread from a needle, even a fine needle, having a diameter of about 0.3 to 0.5 mm.

16. Use of the composition such as defined according to any one of claims 1 to 15, as a vector agent for an active substance, in particular for a biologically, cosmetically or pharmaceutically active substance.

17. Use of the composition according to any one of claims 1 to 15, as filling-in material, in particular for soft or bone tissues, in particular having a stability in vivo at least equal to that of the collagen materials treated with glutaraldehyde.

18. Pharmaceutical composition, characterized in that it comprises a collagen-based composition such as defined in any one of claims 1 to 15.

19. Filling-in-material, characterized in that it was prepared from a collagen-based composition, such as defined in any one of claims 1 to 15.

## Patentansprüche

1. Zusammensetzung auf der Basis von Kollagen, welche leicht injizierbar ist, dadurch gekennzeichnet, daß sie Mikrokapseln aus Atelokollagen oder einer Mischung von Atelokollagen und einem Polysaccharid, insbesondere einem Glykosaminoglykan, in Suspension in einer viskosen biokompatiblen Träger-Lösung umfaßt, wobei die Gesamtheit der Mikrokapseln und der viskosen Träger-Lösung eine Viskosität aufweist, die die Injektion der Zusammensetzung mit einer Nadel ermöglicht.

2. Zusammensetzung nach Anspruch 1, dadurch gekennzeichnet, daß die Träger-Lösung zumindest eine Protein-Substanz, beispielsweise Atelokollagen, und/oder eine Polysaccharid-Substanz umfaßt.

3. Zusammensetzung nach Anspruch 1 oder 2, dadurch gekennzeichnet, daß die Träger-Lösung eine Protein-Substanz, ausgewählt aus Atelokollagen, das vorzugsweise durch eine chemische Behandlung, insbesondere mit Soda, erhalten wird, umfaßt.

4. Zusammensetzung nach einem der Ansprüche 1 bis 3, dadurch gekennzeichnet, daß das Atelokollagen der Mikrokapseln mit einem für die gewünschte Anwendung geeigneten Entnetzungsgrad entnetzt ist.

5. Zusammensetzung nach einem der Ansprüche 2 bis 4, dadurch gekennzeichnet, daß die Polysaccharid-Substanz der Träger-Lösung ausgewählt ist aus einem Glykosaminoglykan, einem Alginat, einem Dextran, einer Cellulose oder einem Cellulose-Derivat, einem Xanthan-Gummi, einem Akaziengummi und ihrer (ihren) Mischung (Mischungen).

6. Zusammensetzung nach einem der Ansprüche 1 bis 5, dadurch gekennzeichnet, daß die Mikrokapseln aus Atelokollagen oder einer Mischung von Atelokollagen und Polysaccharid, die vernetzt sind, hergestellt sind, wobei das Polysaccharid vorzugsweise ausgewählt ist aus einem Glykosaminoglykan mit einer Struktur, ausgewählt aus der Gruppe bestehend aus Chondroitin-4-sulfat, Chondroitin-6-sulfat, Dermatansulfat, Heparansulfat, Keratansulfat sowie Heparin und seinen Derivaten, insbesondere Heparinen mit niedriger Molmasse mit einer Molmasse zwischen ungefähr 2 000 und ungefähr 10 000, und einem Stärkehydrolysat, insbesondere Dextran.

7. Zusammensetzung nach einem der Ansprüche 1 bis 6, dadurch gekennzeichnet, daß das relative Verhältnis Masse/Volumen der Mikrokapseln, bezogen auf die Träger-Lösung, zwischen 0,5 und 15 Masse-% der Mikrokapseln, bezogen auf das Volumen der Träger-Lösung, liegt.

8. Zusammensetzung nach einem der Ansprüche 1 bis 7, dadurch gekennzeichnet, daß das Masseverhältnis in den Stärkehydrolysat-Mikrokapseln, bezogen auf das Atelokollagen, zwischen 10 und 70 % liegt.

9. Zusammensetzung nach einem der Ansprüche 1 bis 8, dadurch gekennzeichnet, daß die Mikrokapseln zumindest eine aktive Substanz, entweder eingekapselt im Inneren der Mikrokapseln oder in der Masse der Wände oder gepfropft auf die innere und/oder äußere Oberfläche, enthält, welche aktive Substanz vorzugsweise eine biologisch, kosmetisch oder pharmazeutisch aktive Substanz ist.

10. Zusammensetzung nach einem der vorhergehenden Ansprüche, dadurch gekennzeichnet, daß die Größe der Mikrokapseln weniger als ungefähr 500 µm beträgt.

11. Zusammensetzung nach einem der Ansprüche 1 bis 10, dadurch gekennzeichnet, daß die Mikrokapseln aus Atelokollagen oder einer Mischung von Atelokollagen und einem Glykosaminoglykan, insbesondere Chondritin-4-sulfat, hergestellt sind, und Hydroxyapatit-Granula enthalten; und die Träger-Lösung aus einem Atelokollagen-Gel, gegebenenfalls gemischt mit einem Glykosaminoglykan, insbesondere Chondroitin-4-sulfat, hergestellt ist.

12. Zusammensetzung nach einem der Ansprüche 1 bis 11, dadurch gekennzeichnet, daß der Kollagen-Gehalt der Zusammensetzung zumindest 7 Masse/Vol-% und vorzugsweise ungefähr 10 Masse/Vol.% beträgt.

13. Zusammensetzung nach einem der Ansprüche 1 bis 12, dadurch gekennzeichnet, daß der Abbau der Mikrokapseln durch die Regulierung des Grenzflächenvernetzungsgrads oder durch einen variablen Entnetzungsgrad von Atelokollagen auf chemischem oder enzymatischem Weg moduliert wird.

14. Zusammensetzung nach einem der Ansprüche 1 bis 13, dadurch gekennzeichnet, daß die viskose biokompatible Träger-Lösung für physiologische pH- und osmotische Druck-Bedingungen geeignet ist, indem sie vorzugsweise einen pH zwischen 6,8 und 7,5 und einen osmotischen Druck zwischen 260 und 320 mOsm/l H₂O aufweist, wobei sie insbesondere mit einem Phosphatpuffer oder einer Mischung von Phosphatpuffer und Natriumchlorid eingestellt wird.

15. Zusammensetzung nach einem der Ansprüche 1 bis 14, dadurch gekennzeichnet, daß die Gesamtheit der Mikrokapseln und der viskosen Träger-Lösung eine Viskosität aufweist, die die Injektion der Zusammensetzung in Form einer kontinuierlichen Faser aus der Öffnung einer feinen Nadel mit einem Durchmesser in der Größenordnung von 0,3 bis 0,5 mm ermöglicht.

16. Verwendung einer Zusammensetzung, wie in einem der Ansprüche 1 bis 15 definiert, als Vektormittel einer aktiven Substanz, insbesondere einer biologisch, kosmetisch oder pharmazeutisch aktiven Substanz.

17. Verwendung einer Zusammensetzung nach einem der Ansprüche 1 bis 15 als Füllmaterial, insbesondere von Weichgeweben oder Knochengeweben, insbesondere mit einer Stabilität in vivo zumindest gleich jener von mit Glutaraldehyd behandelten Kollagen-Materialien.

18. Pharmazeutische Zusammensetzung, dadurch gekennzeichnet, daß sie eine Zusammensetzung auf der Basis von Kollagen, wie in einem der Ansprüche 1 bis 15 definiert, umfaßt.

19. Füllmaterial, dadurch gekennzeichnet, daß es aus einer Zusammensetzung auf der Basis von Kollagen, wie in einem der Ansprüche 1 bis 15 definiert, hergestellt ist.
